**Europäisches Patentamt**

**European Patent Office**

**Office européen des brevets**

(11) Veröffentlichungsnummer : **0 097 281**
**B1**

(12) EUROPÄISCHE PATENTSCHRIFT

(45) Veröffentlichungstag der Patentschrift :
03.04.85

(21) Anmeldenummer : 83105611.4

(22) Anmeldetag : 08.06.83

(51) Int. Cl.⁴ : **C 12 N 11/02, C 12 N 11/04**

(54) Verfahren zur Herstellung eines unlöslichen Biokatalysators.

(30) Priorität : 18.06.82 DE 3222912

(43) Veröffentlichungstag der Anmeldung :
04.01.84 Patentblatt 84/01

(45) Bekanntmachung des Hinweises auf die Patenterteilung : 03.04.85 Patentblatt 85/14

(84) Benannte Vertragsstaaten :
CH DE FR GB LI NL

(56) Entgegenhaltungen :
DE-B- 2 336 829
GB-A- 1 479 097
US-A- 4 337 313

(73) Patentinhaber : BASF Aktiengesellschaft
Carl-Bosch-Strasse 38
D-6700 Ludwigshafen (DE)

(72) Erfinder : Goertz, Hans-Helmut, Dr.
An der Froschlache 23
D-6700 Ludwigshafen (DE)
Erfinder : Marcinowski, Stefan, Dr.
Kopernikusstrasse 49
D-6700 Ludwigshafen (DE)
Erfinder : Sanner, Axel, Dr.
Lorscher Ring 20
D-6710 Frankenthal (DE)

## Beschreibung

Die Erfindung betrifft ein Verfahren zur Herstellung eines unlöslichen Biokatalysators durch Umsetzung eines Amins mit einer Verbindung mit zwei oder mehr (Meth)Acrylamidgruppen in Gegenwart von Enzym oder enzymhaltiger Zellsubstanz.

Für die Verwendung von Biokatalysatoren zur Durchführung von enzymatischen Reaktionen ist in vielen Fällen eine vorherige Immobilisierung wünschenswert. Dadurch wird der Biokatalysator in eine unlösliche Form überführt. In unlöslicher, kleinteiliger Form kann er vorteilhaft für eine kontinuierliche Umsetzung, z. B. in einer Säule, Verwendung finden. Bei der absatzweisen Durchführung der Reaktion unter Aufschlämmen des Katalysators im Reaktionsmedium hat der immobilisierte Biokatalysator den Vorteil der Wiederverwendbarkeit, weil er sich von der Reaktionslösung leicht, z. B. durch Filtrieren oder Dekantieren, abtrennen läßt. In vielen Fällen erhöht die Immobilisierung die Verwendungsdauer des Biokatalysators so erheblich, daß eine wirtschaftliche Nutzung überhaupt erst möglich wird.

Es sind Verfahren zur Immobilisierung von Enzymen und Zellen beschrieben. Ein Teil dieser Verfahren greift auf eine mehr oder weniger physikalische Bindung des Biokatalysators durch einen Träger zurück. Beispielsweise kann das Enzym oder die Zelle durch ionische Kräfte oder durch Adsorptivkräfte am Träger haften, oder es kann in einer Gelmatrix oder einer Membran eingeschlossen sein. In allen diesen Fällen muß aber damit gerechnet werden, daß diese Art der Bindung oder des Einschlusses nicht dauerhaft ist, sondern während der Benutzung der Biokatalysator nach und nach ausgewaschen wird.

Dem stehen Verfahren gegenüber, bei denen der Biokatalysator kovalent an den Träger gebunden ist. Zu unterscheiden sind hier im wesentlichen zwei Verfahrensweisen : Einmal kann zunächst ein Träger hergestellt werden, der aufgrund der Art der Herstellung bereits reaktive funktionelle Gruppen enthält oder nachträglich funktionalisiert oder aktiviert werden kann. Mit einem solchen reaktionsfähigen Träger wird dann der Biokatalysator, zumeist in Form einer Lösung, in Kontakt gebracht, wobei er kovalent gebunden wird. Nachteilig ist hier, daß die Kapazität des Trägers vom Funktionalisierungsgrad bestimmt wird und im allgemeinen sehr gering ist. Außerdem muß bei porösen oder gelartigen Trägern die Porengröße gut auf den zu immobilisierenden Biokatalysator abgestimmt sein. Die Lagerfähigkeit des funktionalisierten Trägers ist zudem meist begrenzt.

Eine andere Möglichkeit zur Erzeugung kovalent verknüpfter unlöslicher (immobilisierter) Biokatalysatoren besteht in der Herstellung eines Trägers in Anwesenheit des Biokatalysators. Dies kann so erfolgen, daß der Biokatalysator zunächst mit einem geeigneten Reagenz funktionalisiert wird und dann als Reaktionspartner bei der Erzeugung des Trägers durch kovalente Bindungen in diesen eingebaut wird. Einfacher ist es, wenn keine vorherige Funktionalisierung des Biokatalysators erforderlich ist, sondern dieser über seine ihm eigenen funktionellen Gruppen, beispielsweise Aminogruppen, während der Erzeugung des Trägers in diesen eingebaut wird. Beispielsweise beschreibt die DE-OS 26 25 471 die Immobilisierung von biologischem Material in Polyurethanschäumen, wobei das reaktionsfähige Polyisocyanat einerseits mit dem biologischen Material reagiert, andererseits den Schaum aufbaut. Wegen oder hohen Reaktivität der Isocyanatpräpolymeren ist in diesem Fall eine Vorbehandlung des trockenen Enzyms mit diesem Präpolymeren angezeigt, was die Anwendbarkeit des Verfahrens stark einschränkt.

DE-OS 28 35 874 beschreibt die Immobilisierung von Zellen oder Zellfragmenten in Mischungen von Epoxiden und geeigneten Härtern, wobei aber die Verwendung eines inerten Hilfspolymeren, das die Formgebung des Katalysators beeinflußt und später wieder entfernt wird, das Verfahren stark kompliziert. Zudem sind die verwendeten Epoxide nicht wasserlöslich, so daß das System ohnehin nur begrenzt verwendungsfähig ist.

DE-OS 29 15 135 offenbart die Herstellung immobilisierter Biokatalysatoren durch Umsetzung des Biokatalysators mit Glutardialdehyd in Anwesenheit eines Polyamins in wäßriger Lösung, wobei der Biokatalysator in das aus Glutardialdehyd und Polyamin entstehende Netzwerk eingebaut wird. Nachteilig ist hier, daß das entstandene Netzwerk keine mechanisch befriedigenden Eigenschaften aufweist. Eine weitere Einschränkung bedeutet die Tatsache, daß das Polyamin einen hohen Prozentsatz an primären Aminogruppen aufweisen muß, da nur primäre, nicht dagegen sekundäre Aminogruppen stabile Vernetzungen mit Glutardialdehyd ergeben.

Der vorliegenden Erfindung lag die Aufgabe zugrunde, ein möglichst einfaches Verfahren zur Immobilisierung von Biokatalysatoren zu entwickeln, das die Nachteile der oben angeführten Verfahren vermeidet.

Gegenstand der Erfindung ist das Verfahren zur Herstellung unlöslicher Biokatalysatoren nach Anspruch 1 und deren Verwendung nach Anspruch 2.

Als biologisch aktive Substanz sind Enzyme oder Zellen geeignet, wobei die Zellen intakt oder denaturiert sein können. Auch gebrochene oder aufgeschlossene oder homogenisierte Zellen oder Zellbruchstücke können eingesetzt werden. Im Fall der Enzyme können Rohpräparationen oder Reinenzyme Verwendung finden. Für das erfindungsgemäße Verfahren geeignete Enzyme sind vorzugsweise Invertase, Glucoseisomerase, Amyloglucosidase und alpha- und beta-Amylase. Ferner sind geeignet : Oxidoreduktasen — wie Alkoholdehydrogenase, Lactatdehydrogenase,

Aminosäureoxidase, Peroxidase, Katalase, Glucoseoxidase, Alkoholoxidase, Succinatdehydrogenase, Glutamatdehydrogenase, Uricase, Phenoloxidase, Catecholoxidase, Monoaminooxidase, Lipoxygenase, Luciferase, Nitratreduktase, Nitritreduktase, Chlorperoxidase, Acetaldehydehydrogenase, Aldehydoxigenase, Diaphorase, Cholesterinoxidase, Glutarthioreduktase, Hydroxysteroiddehydrogenase, Xanthinoxidase, Dopaminhydroxylase, Cytochromoxidase, Monoaminooxidase, Diacetylreduktase, Superoxiddismutase, Limonatdehydrogenase —; Transferasen — wie Polynucleotidphosphorylase, Dextransucrase, Phosphorylase, Carbamatkinase, Aminotransferase, Transaldolase, Methyltransferase, Pyruvatkinase, Carbamoyltransferase, Phosphofructokinase, Dextransynthetase —; Hydrolasen — wie Lipase, Esterase, Lactase, Lysozym, Aminosäureacylase, Penicillinacylase, Cellulase, Urease, Trypsin, Chymotrypsin Glutaminase, Asparaginase, Papain, Ficin, Pepsin, Leucinaminopeptidase, Carboxypeptidase A + B, Maringinase, Bromelain, Subtilisin, Phospholipase, ₁Isoamylase, Cephalosporinamidase, Hydantoinase, Adenosindeaminase, Penicillinase, Maltase, Dextranase, Desoxyribonuclease, Sulfatase, Pullulanase, Phosphatase, alpha-Galactosidase, beta-Glucanase —; Lyasen — wie Tryptophanase, Tyrosindecarboxylase, Oxinitrilase, Phenylalanindecarboxylase, Phenylalaninammoniumlase, Aminosäuredecarboxylase, Pyruvatdecarboxylase, Fumarase, Enolase, Aspartase, Aminolaevulindehydratase, Carboanhydratase —; Isomerasen — wie Aminosäureracemase, Triosephosphatisomerase —; Ligasen — wie Glutathionsynthetase.

Unter einer festen Bindung zwischen biologisch aktiver Substanz und dem Trägermaterial ist hier eine solche (vorzugsweise kovalente) Bindung zu verstehen daß die biologisch aktive Substanz auch in langer Zeit nicht aus dem Träger ausgewaschen wird (unter Umständen abgesehen von einem gewissen Anfangsverlust an biologisch aktiver Substanz, die nur physikalisch gebunden war).

Mit einem unlöslichen Biokatalysator ist ein solcher gemeint, der für die heterogene Katalyse gut geeignet ist, der also aufgrund seiner Unlöslichkeit im (in aller Regel wäßrigen) Reaktionsmedium, seiner Partikelgröße und seiner ausreichend festen Beschaffenheit leicht filtrierbar oder in sonstiger Weise vom Reaktionsmedium abtrennbar ist. Zellen und Zellteile sind zwar ebenfalls unlöslich, sind aber wegen ihrer relativ schlechten Filtrierbarkeit hier nicht gemeint.

Bei Verwendung einer wäßrigen Lösung oder Suspension der biologisch aktiven Substanz (Komponente A) ist die Konzentration unkritisch und kann in weiten Grenzen schwanken. Zweckmäßig läßt man die bei der Gewinnung der Lösung oder Suspension sich ergebende Konzentration unverändert.

Als wasserlösliche Verbindung mit mindestens 2 Methacrylamid- oder vorzugsweise Acrylamid-Gruppen (Komponente B) sind beispielsweise geeignet N,N′-Methylenbisacrylamid, N,N′-Ethylenbisacrylamid oder N,N′-Diacryloylpiperazin sowie die entsprechenden Methacrylverbindungen. Die mechanische Festigkeit der erhaltenen Gele kann verbessert werden, wenn Verbindungen oder Mischungen von Verbindungen mit (durchschnittlich) mehr als 2, vorzugsweise bis 6, reaktionsfähigen Doppelbindungen eingesetzt werden. Solche Verbindungen können beispielsweise erhalten werden, indem man Bisacrylamide der genannten Art in wäßriger Lösung mit einem Unterschuß an Ammoniak oder mindestens einem wasserlöslichen Amin umsetzt, das mehr als 2 an Aminstickstoff gebundene Wasserstoffatome enthält, und zwar in einem solchen Molverhältnis, daß auf jede Amin-NH-Gruppe mehr als 0,5, vorzugsweise 0,7 bis 1 Mol Bisacrylamid kommt. Die so erhaltenen Additionsprodukte zeigen zudem oft eine verbesserte Wasserlöslichkeit, insbesondere, wenn das verwendete Amin (oder mindestens eines von mehreren) hydrophile Gruppen enthält, wie beispielsweise Ethanolamin. Die Wasserlöslichkeit der Komponente B sollte mindestens 3, vorzugsweise mindestens 10 Gew.% bei Raumtemperatur betragen.

Als wasserlösliches Amin mit mindestens 2 an Aminstickstoff gebundenen Wasserstoffatomen (Komponente C) kommen in Betracht Ammoniak, niedere Diamine, z. B. Ethylendiamin, Propylendiamin oder Tetramethylendiamin, oder niedere Polyamine, z. B. Diethylendiamin, Triethylentetramin. Sofern die Komponente B mehr als 2 (Meth)Acrylamid-Gruppen enthält, führen auch primäre Monoamine, wie Ethanolamin, Methylamin, Ethylamin, oder sekundäre Diamine wie N,N′-Dimethylethylendiamin, zur Vernetzung. Es genügt, wenn die Summe der Zahl der (Meth)Acrylamid-Gruppen pro Molekül der Komponente B und der Zahl der NH-Gruppen pro Molekül der Komponente B und der Zahl der NH-Gruppen pro Molekül der Komponente C größer als 4 ist, also mindestens etwa 4,1, besser aber 4,5 oder 5 oder vorzugsweise noch größer. Ganz besonders geeignete Aminoverbindungen sind polymere Verbindungen wie Polyethylenimin, linear oder verzweigt, oder Polyvinylamin. Diese Amine bewirken eine deutliche Verbesserung der mechanischen Stabilität des immobilisierten Biokatalysators.

Die Herstellung des immobilisierten Biokatalysators erfolgt durch Mischen der 3 Komponenten A, B und C. Die beiden letztgenannten Komponenten werden zweckmäßig als wäßrige Lösungen eingesetzt, wobei die Konzentrationen bevorzugt zwischen 20 und 50 Gew.% liegen. Das molare Verhältnis von (Meth)Acrylamidgruppen der Komponente B zu Amin-Wasserstoffatomen der Komponente C liegt vorzugsweise zwischen 1 : 0,5 und 1 : 20. Vorzugsweise wird die biologisch aktive Substanz (Komponente A), die zweckmäßig als Lösung oder Suspension, aber auch als Pulver eingesetzt werden kann, zunächst mit der Lösung der Komponente B gemischt und erst nach einiger Zeit (1 Minute bis 2 Stunden) die Lösung der Komponente C zugefügt, jedoch ist je nach Fall auch eine andere Reihenfolge möglich.

Die Komponente C kann in neutralisierter, teilneutralisierter oder nicht neutralisierter Form zugegeben werden, was im wesentlichen durch die pH-Wert-Stabilität der biologisch aktiven Substanz bestimmt wird. Wird die Aminoverbindung in nicht neutralisierter Form eingesetzt, so ist der pH-Wert in der Reaktionsmischung im allgemeinen recht hoch (z. B. bei 9 bis 11). Die Gelbildung läuft unter diesen Bedingungen relativ schnell ab. Auf viele Enzyme wirken diese hohen pH-Werte jedoch denaturierend, so daß eine Neutralisierung der Komponente C empfehlenswert ist. Ein wesentlicher Vorteil des hier beschriebenen Verfahrens ist, daß die Gelbildung, wenn auch verlangsamt, auch bei relativ niedrigen pH-Werten (z. B. 5 bis 6) stattfindet.

Sofern die thermische Stabilität der verwendeten biologisch aktiven Substanz es zuläßt, kann die Gelbildung bei erhöhter Temperatur, z. B. bei 50 °C vorgenommen werden. Sie kann so erheblich beschleunigt werden. Die Anwendung von Temperaturen unter Raumtemperatur bringt keine Vorteile. Die Gelbildung kann eine Stunde bis 4 Tage, vorzugsweise 2 bis 8 Stunden in Anspruch nehmen. Längere Zeiten sind aus wirtschaftlichen Gründen kaum noch von Interesse.

Das Gel kann nach gängigen Methoden zerkleinert werden, z. B. kann man es durch ein Sieb drücken, zerschneiden, extrudieren. Es kann vor oder nach dem Zerkleinern getrocknet, gegebenenfalls gefriergetrocknet werden. Als günstige Partikelgrößen haben sich Teilchendurchmesser im Bereich von 0,1 bis 5 mm, bewährt.

Um bei der Verfestigung der Mischung von vornherein ein kleinteiliges Produkt zu erhalten, kann man die Mischung unter Rühren in einem inerten, mit Wasser nicht mischbaren Lösungsmittel, eventuell unter Zuhilfenahme eines üblichen Suspensionhilfsmittels, suspendieren. Die Mischung verfestigt sich dann in Form von Perlen, wobei deren Größe im obengenannten Rahmen (0,1 bis 5 mm Durchmesser) in üblicher Weise durch geeignete Wahl von Gefäß, Rührer und Rührgeschwindigkeit beeinflußt werden kann. Als mit Wasser nicht mischbare Lösungsmittel kommen insbesondere in Frage : aliphatische, cycloaliphatische und aromatische Kohlenwasserstoffe wie z. B. Hexan, Cyclohexan und Toluol sowie chlorierte Kohlenwasserstoffe wie 1,1,1-Trichlorethan. Es ist zweckmäßig, zur besseren Suspendierung der wäßrigen Phase Suspensionshilfsmittel zuzusetzen. Hierzu sind Substanzen geeignet, wie sie bei der umgekehrten Suspensionspolymerisation Verwendung finden, z. B. Sorbitanester. Nach der Verfestigung wird der Katalysator abfiltriert und gründlich mit Wasser oder einer wäßrigen Pufferlösung gewaschen. Eine vorherige kurze Waschung mit einem wasserlöslichen organischen Lösungsmittel wie z. B. Aceton oder Alkohol kann vorteilhaft sein, ist jedoch nicht in jedem Fall notwendig.

Kritisch bei der Immobilisierung von Biokatalysatoren ist zum einen die möglichst weitgehende Erhaltung der biologischen Aktivität während des Immobilisierungsprozesses und zum andern die Stabilität hinsichtlich der Wirkung des so erhaltenen Katalysators bei der Lagerung und insbesondere unter den für die beabsichtigten Umsetzungen erforderlichen Reaktionsbedingungen (hauptsächlich Temperatur und pH-Wert). Außerdem spielt die mechanische Festigkeit eine gewisse Rolle. Bei vielen Biokatalysatoren führt keine der bekannten Immobilisierungsmethoden zu einem befriedigenden Ergebnis. In vielen Fällen ist die Methode aufwendig, in anderen Fällen ist die Anwendung auf einen engen pH-Bereich beschränkt, in der Mehrzahl aller Fälle ist die nach der Immobilisierung verbleibende Restaktivität gering oder kurzlebig. Das erfindungsgemäße Verfahren ist den bekannten oft in einer oder mehrfacher Hinsicht überlegen.

Beispiel 1

25 mg β-Fructosidase wurden in 5 ml 0,05 M Natriumacetatlösung vom pH 5,3 gelöst. Zu dieser Lösung wurden nacheinander 5 ml einer 20 %igen Lösung eines Additionsproduktes von 4,7,10-Trioxatridecan-1,13-diamin und N,N'-Methylenbisacrylamid (molares Verhältnis 1 : 4) und 5 ml einer 25 %igen wäßrigen Lösung von Polyethylenimin (MW 30 000 ; mit Salzsäure auf pH 6,0 eingestellt) unter Rühren zugefügt. Diese Mischung wurde zwei Tage bei Raumtemperatur belassen. Das erhaltene feste Gel wurde durch ein Sieb gedrückt (Partikelgröße unter 500 μm) und mit 0,05 M Natriumacetatlösung vom pH 5,3 gewaschen. 2,5 g Immobilisat (entsprechend 4,2 mg eingesetztem Enzym) wurden 30 min bei 30 % mit 50 ml einer 30 %igen Saccharose-Lösung inkubiert. Der Umsatz wurde polarimetrisch zu 30 % bestimmt. Dies entspricht einer Restaktivität von 70 %, bezogen auf eine entsprechende Menge freies Enzym.

Beispiel 2

5 g Trockenhefe wurden in 50 ml 0,9 %iger Kochsalzlösung suspendiert und für 1 Stunde belassen. Danach wurde zentrifugiert und der Überstand abdekantiert. Die feuchte Hefe wurde mit 25 g einer 40 %igen wäßrigen Lösung eines Additionsproduktes von N,N'-Methylenbisacrylamid, Ethanolamin und Ethylendiamin (Molverhältnis 8 : 4 : 1) und 25 g einer 25 %igen wäßrigen Lösung von Polyethylenimin (MW 30 000 ; mit Salzsäure auf pH 6,0 eingestellt) vermischt und 3 Tage bei Raumtemperatur belassen. Das erhaltene feste Gel wurde zerkleinert (Partikelgröße < 500 μm). 16,2 g dieses Gels (entsprechend 1,25 g Trockenhefe) wurden in eine Säule gefüllt und bei 30 °C mit einer Fließgeschwindigkeit von 20 ml/h eine 35 %ige Saccharose-Lösung, die durch 0,02 Mol/l Natriumacetat auf pH 5,3 eingestellt war, darübergeleitet. Der Umsetzungsgrad wurde polarimetrisch bestimmt und betrug nach 2 Tagen 88,5 %, nach 16 Tagen 87 %, nach 28 Tagen 84,5 %.

Beispiel 3

1 g Trockenhefe wurde 1 Stunde in 10 ml 0,9 %iger Kochsalzlösung suspendiert. Danach wurde abzentrifugiert und der Überstand dekantiert. Die feuchte Hefe (3,0 g) wurde mit 5 g einer 40 %igen wäßrigen Lösung eines Additionsproduktes von N,N'-Methylenbisacrylamid, Ethanolamin und Ethylendiamin Molverhältnis 8 : 4 : 1) und 5 g einer 25 %igen wäßrigen Lösung von Polyethylenimin (MW 30 000 ; mit Salzsäure auf pH 6,0 eingestellt) vermischt. Die so erhaltene Mischung wurde in 100 ml Cyclohexan suspendiert (Suspensionshilfsmittel : 100 mg Schutzkolloid A nach DE-OS 26 34 486) und 3 Tage bei Raumtemperatur gerührt. Danach wurde abfiltriert und der verfestigte Katalysator mit 1 l, 0,05 M Natriumacetatpuffer pH 5,3 gewaschen.

Zur Ermittlung der Invertase-Aktivität wurden 2,6 g Katalysator (entsprechend 0,2 g Trockenhefe) 90 min bei 30 °C in 50 ml 30 %iger Saccharoselösung (pH 5,3) geschüttelt. Der Umsatz wurde polarimetrisch zu 15 % bestimmt. Dies entspricht einer Invertase-Restaktivität von 50 %.

Beispiel 4

4 mg α-Amylase (130 Units/mg) wurden in 2 g einer 20 %igen wäßrigen Lösung eines Additionsproduktes von 4,7,10-Trioxatridecan-1,13-diamin und N,N'-Methylenbisacrylamid (Molverhältnis 1 : 4) aufgelöst und die erhaltene Lösung mit 2 g einer 25 %igen Lösung von Polyethylenimin (MW 30 000 ; mit Salzsäure auf pH 6,0 eingestellt) gemischt. Nach 3 tägigem Stehen bei Raumtemperatur wurde das entstandene Gel durch ein Sieb gedrückt (Maschenweite 500 µm) und danach mit 100 ml 0,05 M wäßriger Natriumacetatpufferlösung vom pH 6,0 gewaschen. Zur Aktivitätsbestimmung wurde 1 g des Gels bei 30 °C 30 min in 50 ml Lösung von 5 g Stärke nach Zulkowsky (Merck) in 0,016 M Natriumacetatpufferlösung (pH 6,0) geschüttelt. Die entstandenen Oligosaccharide wurden mit 3,5-Dinitrosalicylsäure nach P. Berenfeld (Methods in Enzymology, Vol. I, 149, Academic Press, 1955) bestimmt. Aktivität des Gels : 57,2 Units/g (immobilisierungsausbeute 44 %).

Beispiel 5

Analog Beispiel 4 wurden 8 mg β-Amylase (28 Units/mg) in 4 g Gel immobilisiert. Das zerkleinerte Gel wurde mit 100 ml 0,05 M wäßriger Natriumacetatpufferlösung vom pH 4,6 gewaschen. Zur Aktivitätsbestimmung wurde 1 g Gel in 50 ml Lösung von 5 g Stärke nach Zulkowsky (Merck) in 0,05 M Natriumacetatpufferlösung (pH 4,8) geschüttelt. Die gebildete Maltose wurde mit 3,5-Dinitrosalicylsäure nach P. Berenfeld (s. o.) bestimmt. Aktivität des Gels : 38,6 Units/g (Immobilisierungsausbeute 69 %).

Beispiel 6

Analog Beispiel 5 wurde Amyloglucosidase immobilisiert. Immobilisierungsausbeute 38 %.

**Ansprüche**

1. Verfahren zur Herstellung eines unlöslichen Biokatalysators aus einem synthetischen Trägermaterial und einer fest darangebundenen biologisch aktiven Substanz, dadurch gekennzeichnet, daß man folgende Komponenten in wäßriger Lösung oder Suspension miteinander mischt :

A eine biologische aktive Substanz,

B mindestens eine wasserlösliche Verbindung mit mindestens 2 Acrylamid- oder Methacrylamid-Gruppen und

C mindestens ein wasserlösliches aliphatisches Amin mit mindestens 2 an Aminstickstoff gebundenen Wasserstoffatomen,

mit der Maßgabe, daß die Summe der (durchschnittlichen) Zahl der (Meth)Acrylamid-Gruppen von Komponente B und der (durchschnittlichen) Zahl der an Aminstickstoff gebundenen Wasserstoffatome von Komponente C, jeweils pro Molekül, größer als 4 ist,

und daß man entweder das im Lauf von einer Stunde bis zu 4 Tagen bei Temperaturen oberhalb 15 °C entstandene Gel zerkleinert, oder daß man die wäßrige Mischung in einem mit Wasser nicht mischbaren Lösungsmittel bis zur Verfestigung in Form von Tröpfchen suspendiert hält und den so entstandenen kleinteiligen Katalysator vom Lösungsmittel abtrennt.

2. Verwendung eines nach Anspruch 1 hergestellten unlöslichen Biokatalysators zur heterogenen Enzymkatalyse.

**Claims**

1. A process for the preparation of an insoluble biocatalyst from a synthetic carrier and a biologically active substance firmly bonded to the carrier, wherein the following components, in aqueous solution or suspension, are mixed together :

A a biologically active substance,

B one or more water-soluble compounds possessing 2 or more acrylamide or methacrylamide groups, and

C one or more water-soluble aliphatic amines possessing 2 or more hydrogen atoms bonded to the amine nitrogen,

with the proviso that the sum of the (average) number of acrylamide or methacrylamide groups of component B and the (average) number of hydrogen atoms bonded to the amine nitrogen of component C, in each case per molecule, is greater than 4,

and either the gel formed in the course of from one hour to 4 days at above 15 °C is comminuted, or the aqueous mixture is kept in suspension in a water-immiscible solvent until solidification to droplets takes place and the resulting finely divided catalyst is isolated from the solvent.

2. The use of an insoluble biocatalyst, prepared as claimed in claim 1, for the heterogeneous enzyme catalysis.

## Revendications

1. Procédé de préparation d'un biocatalyseur insoluble, à partir d'un matériau support synthétique et d'une substance biologiquement active, solidement liée à ce support, caractérisé par le fait que l'on mélange les uns aux autres, en solution aqueuse ou en suspension, les composants suivants :

    A une substance biologiquement active,

    B au moins un composé soluble dans l'eau, ayant au moins 2 groupes acrylamide ou méthacrylamide, et

    C au moins une amine aliphatique soluble dans l'eau ayant au moins 2 atomes d'hydrogène liés à l'azote d'amine,

avec la condition que la somme du nombre (moyen) des groupes (méth)acrylamide du composant B et du nombre (moyen) des atomes d'hydrogène du composant C, liés à l'azote d'amine, pour chacun par molécule, soit supérieur à 4,

et, soit l'on triture le gel obtenu, à des températures supérieures à 15 °C pendant une durée d'une heure à 4 jours, soit l'on maintient en suspension le mélange aqueux dans un solvant non miscible avec l'eau, jusqu'à solidification sous forme de gouttelettes et l'on sépare du solvant le catalyseur finement divisé, ainsi obtenu.

2. Utilisation d'un biocatalyseur insoluble préparé selon la revendication 1, pour la catalyse enzymatique hétérogène.